Europäisches Patentamt

(19) European Patent Office

Office européen des brevets

(11) Publication number: **0 237 194 B1**

## (12) EUROPEAN PATENT SPECIFICATION

(45) Date of publication of patent specification:
**17.04.91 Bulletin 91/16**

(51) Int. Cl.⁵: **A61F 15/00, B65D 81/14**

(21) Application number: **87301193.6**

(22) Date of filing: **12.02.87**

(54) Wrapping means and method of wrapping.

(30) Priority: **07.03.86 US 837582**
**25.06.86 US 878173**

(43) Date of publication of application:
**16.09.87 Bulletin 87/38**

(45) Publication of the grant of the patent:
**17.04.91 Bulletin 91/16**

(84) Designated Contracting States:
**DE FR GB IT**

(56) References cited:
**GB-A- 2 163 356**
**US-A- 4 286 603**

(73) Proprietor: **Command Automation Inc.,**
**25 West Broward Boulevard**
**Fort Lauderdale Florida 33301 (US)**

(72) Inventor: **Simjian, Luther G.**
**1750 South Ocean Lane**
**Fort Lauderdale Florida, 33316 (US)**

(74) Representative: **Symonds, John Francis et al**
**Brookes & Martin High Holborn House 52/54,**
**High Holborn**
**London WC1V 6SE (GB)**

## Description

This invention broadly relates to a wrapping means and a method of wrapping.

Most surgical procedures require the application of a medical bandage over the affected area in order to protect the wound, incision, exposed body tissue, etc, and to promote healing. Generally, a bandage of cotton, gauze or other suitable medium is used. A moisture resistant material may form the outer cover layer. When an orthopaedic procedure is involved, frequently a plaster cast is required to immobilize a limb. It is common practice to use a thin layer of gauze over which the plaster cast is applied.

Plaster casts and bandages which are applied too tightly over the skin or wound not only present problems in promoting sound healing, but also are uncomfortable to the patient. Particularly in the field of cosmetic facial surgery, it has been reported that bandages which are too tightly applied cause undesirable side effects, such as severe ear aches. It is then necessary to promptly remove such a bandage and re-apply it upon the patient. In connection with the protection of inanimate articles, such as works of art during transportation, the article may be damaged if a wrapping is applied too tightly.

United States patent No. 4 286 603 proposes the incorporation of a capsule containing dye into a plaster cast. The indication of excess pressure provided by such a capsule is limited in area and may be overlooked. Also, the incorporation of a capsule according to this patent into a bandage of cotton or gauze is difficult.

Moreover, this previously proposed arrangement does not allow for an indication to be given of a lesser degree of tightness, e.g. when the plaster cast or bandage has been applied correctly, because the staining of a correctly applied bandage with the contents of such a capsule would be unacceptable.

The present invention addresses itself to this problem of providing an indication of excess pressure which is of general application and will give an indication over an extended area, and can be used to indicate varying degrees of tightness. Herein the expression "wrapping means" includes bandages and plaster casts for use in medicine, surgery and first aid, as well as wrappings for use on inanimate articles.

According to one aspect of the invention, a wrapping means comprising wrapping material and pressure responsive means for providing an indication of the tightness of the wrapping material, is characterised in that the pressure responsive means comprises a plurality of strips each of which is rupturable at a predetermined pressure different from that of the other strip or strips, each strip containing a substance in an encapsulated form which is released upon rupture of the strip, and the substance associated with one strip differs from the substance associated with the other strip or strips.

The strips may incorporate substances which when exposed upon rupture of the strips upon the application of predetermined pressures, provide a visual or odoriferous indication of the pressure. Thus the substances may be a fluid (liquid or gas) in encapsulated form. If the wrapping means is too tight when applied to the affected area, one or more of the strips ruptures and the fluid (liquid or gas) is released, and penetrates into the wrapping means to thereby provide a visual or odoriferous indication of a wrapping means deemed too tight. The strip or sheet may contain a dye which will stain the wrapping means to provide a visual indication. If the strip or sheet contains an odorous gas, the released gas will penetrate through the wrapping material and provide an indication to the olfactory organs.

The strips may contain dyes of different colours. Therefore, the user will obtain a more detailed indication of the prevailing pressure condition.

According to another aspect of the invention there is provided a method of wrapping an inanimate article including disposing between the article (10) to be wrapped and the outer surface (22) of the wrapping (20) a pressure responsive means (16) containing an encapsulated substance, said means rupturing in response to a predetermined pressure applied thereupon to release the substance to provide an indication of the predetermined pressure, characterised in that the pressure responsive means is in the form of a plurality of strips (16A, 16B) disposed between the article (10) and the outer surface (22) of the wrapping (20), each strip containing a substance in an encapsulated form which is released upon rupture of the respective strip, and the substance associated with one strip differing from the substance associated with the other strip or strips.

In the drawings :
FIGURE 1 is a sectional view of wrapping means in accordance with the invention, and
FIGURE 2 is a plan view of the rupturable medium containing encapsulated dye.

The present invention makes use of encapsulating techniques which are already known in quite different fields, but which have not been applied to bandaging or other wrapping operations.

Thus, for example, the use of encapsulated dye material in a medium which is rupturable in response to pressure to provide a visual indication is well known. A typical product of this type is the carbonless paper in which the reverse side of the paper is provided with micro-encapsulated dye. When writing on such paper, the medium is ruptured and dye material is released and transferred to an underlying sheet of paper. In this manner, the use of carbon paper is eliminated.

It is also known for an odoriferous gaseous substance to be encapsulated, on a sheet of paper, the gas being released responsive to scratching the sheet in order to provide a sample of a perfume, or for teaching persons to recognise the smell of escaping town or natural gas, for example. It is further known for air to be encapsulated in plastic sheeting to provide a cushioning material used for packaging delicate articles. While the plastics material used for encapsulating air is of such thickness as to be burst resistant, it will be within the skill of the technician to provide such material which is thin enough to rupture under the range of pressures encountered when a bandage or other wrapping means is applied too tightly and release the encapsulated volume of air.

As will be noted from the above the encapsulating of a liquid dye and of gas in a rupturable medium is well known.

Referring now to the figures there is shown a portion 10 of a human body upon which a bandage in accordance with the invention is applied. The bandage, in a typical embodiment, comprises a thin layer of gauze 12 superposed on the body portion 10, a liquid barrier layer 14, such as a thin layer of plastic film, a plurality of rupturable strips 16A and 16B containing encapsulated liquid dye 18A and 18B and a final bandage material 20 having an exposed outer surface 22.

The strips 16A and 16B may comprise thin plastic film, or gelatin material or other suitable substance adapted to contain the dyes 18A and 18B in an encapsulated manner. The dyes are preferably vegetable base dyes, but can be dyes made from other substances. The dyes may be encapsulated in liquid or semi-liquid form, but are designed to flow freely when released,

The strip 16A is arranged to rupture at a pressure lower than that at which the strip 16B ruptures. Upon application of pressure the strips will rupture at one or more locations, thereby releasing encapsulated dye 10. The dye, generally of a contrasting colour, will penetrate into the bandage layer 20, causing it to be stained and thereby providing a visual indication in the form of a stain at the exposed surface 22. The barrier layer 14 prevents the dye from running upon the body portion 10 which may contain an open wound. If this is of no concern, the barrier layer 14 can be omitted.

Strip 16A contains encapsulated dye 18A of a first colour and strip 16B contains dye 18B of a second colour. If the medium of strip 16A ruptures responsive to a lower pressure than that of medium of strip 16B, the physician will receive an indication of the severity of the problem, or whether the pressure is just right if the strip 16A is made to rupture responsive to normal pressure conditions. It will be apparent that some experimentation will be required as different portions of the human body can accept different predetermined levels of pressure.

If the bandage is readily stained responsive to the flow of liquid and such staining is readily discernible upon visual inspection, the encapsulated liquid can be colourless.

In an alternative embodiment of the present invention the encapsulated fluid comprises an odoriferous gaseous substance, or a substance which gives off a perfume when exposed to the atmosphere. The release of the odoriferous gas or perfume can be discerned by the attending physician. Thus, the strips 16A and 16B may contain gaseous substances with different odours, such as sweet and sour smelling substances, for discerning which of the strips has been ruptured. In a still further alternative embodiment, one strip may contain a liquid while the other strip, rupturable at a different pressure, may contain an odoriferous gas.

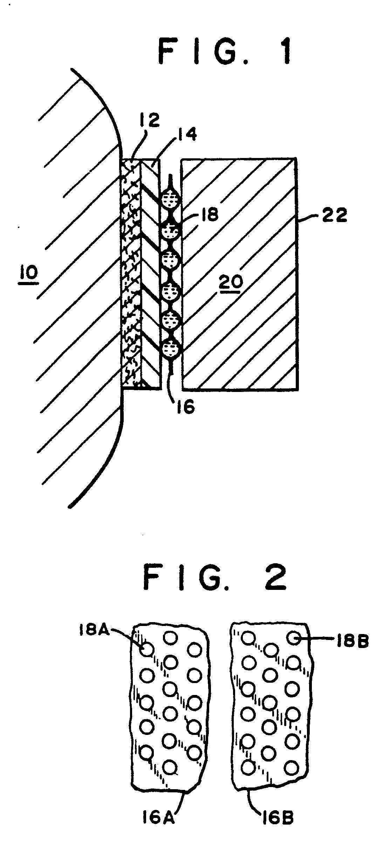

As shown in Figures 1 and 2, the medium 16 in sheet or strip form may comprise a part of a partially assembled bandage or, alternatively, the strips 16A, 16B may be furnished separately and inserted prior to use.

While it is believed that the above described wrapping means is primarily suited for medical and first-aid purposes, it should be understood that it may also be useful in other applications, particularly in the training of first-aid and medical personnel, and where a delicate article needs to be protected during use, shipping or other critical situations

## Claims

1. A wrapping means comprising wrapping material (12) and pressure responsive means (16) for providing an indication of the tightness of the wrapping material, characterised in that the pressure responsive means comprises a plurality of strips (16A, 16B) each of which is rupturable at a predetermined pressure different from that of the other strip or strips, each strip containing a substance in an encapsulated form which is release upon rupture of the strip, and the substance associated with one strip differing from the substance associated with the other strip or strips.

2. A wrapping means according to claim 1, characterised in that the strips (16A, 16B) contain dyes of different colours.

3. A wrapping means according to claim 1, characterised in that the strips (16A, 16B) contain gaseous substances having different odours.

4. A wrapping means according to claim 1, characterised in that one strip contains a dye and another strip contains an odoriferous gaseous substance.

5. A wrapping means according to any preceding claim, characterised in that the wrapping means is a bandage.

6. A method of wrapping an inanimate article including disposing between the article (10) to be wrapped and the outer surface (22) of the wrapping (20) a pressure responsive means (16) containing an encapsulated substance, said means rupturing in response to a predetermined pressure applied thereupon to release the substance to provide an indication of the predetermined pressure, characterised in that the pressure responsive means is in the form of a plurality of strips (16A, 16B) disposed between the article (10) and the outer surface (22) of the wrapping (20), each strip containing a substance in an encapsulated form which is released upon rupture of the respective strip, and the substance associated with one strip differing from the substance associated with the other strip or strips.

## Ansprüche

1. Verpackungsmittel mit Verpackungsmaterial (12) sowie mit einem auf Druck ansprechenden Mittel (16) zum Erzeugen einer Anzeige der Dichtheit des Verpackungsmateriales, dadurch gekennzeichnet, daß das auf Druck ansprechende Mittel eine Mehrzahl von Streifen (16A, 16B) aufweist, deren jeder bei einem vorgegebenen Druck abreißt, welcher verschieden von jenem des anderen Streifens oder der anderen Streifen ist, daß jeder Streifen eine Substanz in gekapselter Form enthält, die beim Abreißen des Streifens freigegeben wird, und daß die einem Streifen zugeordnete Substanz von der Substanz abweicht, die dem anderen Streifen, bzw. den anderen Streifen zugeordnet ist.

2. Verpackungsmittel nach Anspruch 1, dadurch gekennzeichnet, daß die Streifen (16A, 16B) Farbstoffe unterschiedlicher Farben enthalten.

3. Verpackungsmittel nach Anspruch 1, dadurch gekennzeichnet, daß die Streifen (16A, 16B) gasförmige Substanzen unterschiedlicher Gerüche enthalten.

4. Verpackungsmittel gemäß Anspruch 1, dadurch gekennzeichnet, daß ein Streifen einen Farbstoff und ein anderer Streifen eine geruchabgebende gasförmige Substanz enthält.

5. Verpackungsmittel nach einem der vorausgegangenen Ansprüche, dadurch gekennzeichnet, daß das Verpackungsmittel eine Bandage ist.

6. Verfahren zum Verpacken eines toten Gegenstandes, wobei zwischen den einzupackenden Gegenstand und die Außenfläche (22) der Verpackung (20) ein auf Druck ansprechendes Mittel (16) eingelegt wird, das eine eingekapselte Substanz enthält, und wobei das Mittel in Abhängigkeit von einem vorgegebenen, hierauf aufgebrachten Druck reißt, um die Substanz freizugeben und eine Anzeige des vorgegebenen Druckes zu liefern, dadurch gekennzeichnet, daß das auf Druck ansprechende Mittel in Gestalt einer Mehrzahl von Streifen (16A, 16B) zwischen den Gegenstand (10) und die Außenfläche (22) der Verpackung (20) eingelegt wird, wobei jeder Streifen eine Substanz in gekapselter Form enthält, die bei Abriß des betreffenden Streifens, freigesetzt wird, und wobei die Substanz, die einem Streifen zugeordnet ist, von der Substanz abweicht, die dem bzw. den anderen Streifen zugeordnet ist.

## Revendications

1. Moyen d'enveloppement comprenant de la matière d'enveloppement (12) et un moyen sensible à la pression (16) pour fournir une indication du serrage de la matière d'enveloppement, caractérisé en ce que le moyen sensible à la pression comprend une pluralité de bandelettes (16A, 16B) dont chacune est susceptible d'être rompue à une pression déterminée différente de celle de l'autre bandelette ou des autres bandelettes, chaque bandelette contenant une substance sous une forme encapsulée qui est libérée sous l'effet de la rupture de la bandelette, et la substance associée à une bandelette étant différente de la substance associée à l'autre bandelette ou aux autres bandelettes.

2. Moyen d'enveloppement selon la revendication 1, caractérisé en ce que les bandelettes (16A, 16B) contiennent des colorants de couleurs différentes.

3. Moyen d'enveloppement selon la revendication 1, caractérisé en ce que les bandelettes (16A, 16B) contiennent des substances gazeuses ayant des odeurs différentes.

4. Moyen d'enveloppement selon la revendication 1, caractérisé en ce qu'une bandelette contient un colorant et qu'une autre bandelette contient une substance gazeuse odorante.

5. Moyen d'enveloppement selon l'une quelconque des revendications précédentes, caractérisé en ce que le moyen d'enveloppement est un pansement.

6. Procédé d'enveloppement d'un article inanimé comprenant le fait de disposer entre l'article (10) à envelopper et la surface externe (22) du moyen d'enveloppement (20) un moyen (16) sensible à la pression contenant une substance encapsulee, ledit moyen (16) se rompant sous l'effet d'une pression prédéterminée qui lui est appliquée, pour dégager la substance afin de fournir une indication de la pression prédéterminée, caractérisé en ce que le moyen sensible à la pression est sous la forme d'une pluralité de bandelettes (16A, 16B) disposées entre l'article (10) et la surface externe (22) du moyen d'enveloppement (20), chaque bandelette contenant une substance sous forme encapsulée qui est dégagée lors de la rupture de la bandelette respective, et en ce que la substance associée à une bandelette est différente de la substance associée à l'autre bandelette ou aux autres bandelettes.

# FIG. 1

# FIG. 2